# EUROPEAN PATENT SPECIFICATION

(11) **EP 4 199 849 B1**
(45) Date of publication and mention of the grant of the patent: **10.07.2024**
(21) Application number: 21717605.6
(22) Date of filing: 04.03.2021
(51) Int. Cl.: A61B 18/24, A61B 18/26

(54) **FASTER RISE TIME PULSE SHAPING OF PLASMA GENERATED PRESSURE WAVES FOR DISRUPTION OF VASCULAR CALCIUM**
IMPULSFORMUNG VON PLASMAERZEUGTEN DRUCKWELLEN MIT SCHNELLERER ANSTIEGSZEIT ZUR UNTERBRECHUNG VON VASKULÄREM CALCIUM
MISE EN FORME D'IMPULSION DE TEMPS DE MONTÉE PLUS RAPIDE D'ONDES DE PRESSION GÉNÉRÉES PAR PLASMA POUR DÉSAGRÉGATION DE CALCIUM VASCULAIRE

(30) Priority: 19.08.2020 US 202063067780 P; 03.03.2021 US 202117190913
(43) Date of publication of application: 28.06.2023
(73) Proprietor: Bolt Medical, Inc., Carlsbad, CA 92008 (US)
(72) Inventor: BACHER, Gerald David, Carlsbad, CA 92008 (US); COOK, Christopher A., Laguna Niguel, CA 92677 (US)
(74) Representative: Potter Clarkson
(86) International application number: PCT/US2021/020934
(87) International publication number: WO 2022/039783

(56) References cited:
- US-A1- 2007 299 392
- US-A1- 2015 100 048
- US-A1- 2017 265 942
- US-A1- 2020 129 195

## Description

### BACKGROUND

Vascular lesions within and adjacent to vessels in the body can be associated with an increased risk for major adverse events, such as myocardial infarction, embolism, deep vein thrombosis, stroke, and the like. Severe vascular lesions can be difficult to treat and achieve patency for a physician in a clinical setting.

Vascular lesions may be treated using interventions such as drug therapy, balloon angioplasty, atherectomy, stent placement, vascular graft bypass, to name
a few. Such interventions may not always be ideal or may require subsequent treatment to address the lesion.

Using optical fiber delivery of laser pulses to generate high-pressure impulses on the lesions is one way to attempt to treat the lesions. Creation of a plasma via optical breakdown of an aqueous solution typically requires a significant amount of energy in a short amount of time upon which it is converted into a therapeutic bubble and/or a therapeutic pressure wave. With sufficiently high energy and short pulse durations, there is potential to damage a fiber proximal end and/or a fiber distal end of the optical fiber used to deliver light energy to generate the plasma. This damage to the fiber proximal end of the optical fiber usually manifests as surface damage. The amount of energy that can be transmitted through the fiber is limited by the peak intensity of the pulse on the proximal surface of the fiber. Additionally, for pulses with an approximately Gaussian temporal shape, the peak pressure generated by the optical pulse is approximately proportional to the peak intensity of the pulse.

Further, creation of the plasma near the fiber distal end of the optical fiber as in the case of aqueous optical breakdown as one method for an intravascular lithotripsy catheter has the potential for self-damage due to its proximity to the plasma creation and/or the pressure wave, high plasma temperatures, and waterjet from collapse of the bubble, as non-exclusive examples.

US 2017/265942 A1 relates generally to the use of medical devices for the treatment of vascular conditions. In particular, the present disclosure provides materials and methods for using laser-induced pressure waves to disrupt vascular blockages and to deliver therapeutic agents to the blockage area.

US 2007/299392 A1 relates generally to a balloon for injecting material into a wall of a hollow organ of a human, comprising: an expandable balloon body having a surface and having an axis; at least one predefined ejection port on said body adapted for ejection of fluid therefrom, in a transaxial direction; and an impulse source configured for and adapted to eject material out of said point at a velocity and shape suitable for mechanically penetrating tissue adjacent said port.

US 2020/129195 A1 is directed toward a photoacoustic catheter for placement within a blood vessel having a vessel wall. The photoacoustic catheter can be used for treating a calcified lesion within or adjacent to the vessel wall.

A further vascular catheter for laser lithotripsy is disclosed in US2015/0100048 A1.

### SUMMARY

The invention is defined in the appended set of claims.

The present invention is directed toward a catheter system for treating a treatment site within or adjacent to a vessel wall. In various embodiments, the catheter system includes an inflatable balloon, an optical fiber and a laser. The optical fiber can have a distal end that is positioned within the inflatable balloon. The optical fiber can be configured to receive an energy pulse so that the optical fiber emits light energy in a direction away from the optical fiber to generate a plasma pulse within the inflatable balloon. The laser can include (i) a seed source that is configured to emit a seed pulse, and (ii) an amplifier that is configured to increase energy of the seed pulse so that the laser generates the energy pulse that is received by the optical fiber. In certain embodiments, the energy pulse can have a waveform with a duration T, a minimum power P₀, a peak power P_{P}, and a time from P₀ to P_{P} equal to T_{P}, wherein T_{P} is not greater than 40%, 30%, 25%, 20%, 10% or 5% of T.

In various embodiments, T can be within the range of greater than 50 ns and less than 3 µs, greater than 100 ns and less than 2 µs, greater than 200 ns and less than 1 µs, greater than 300 ns and less than 800 ns, or greater than 400 ns and less than 600 ns.

In some embodiments, T_{P} can be within the range of greater than 50 ns and less than 3 µs, greater than 100 ns and less than 2 µs, greater than 200 ns and less than 1 µs, or greater than 300 ns and less than 800 ns. P_{P} is within the range of greater than 50 kW and less than 1000 kW, preferably greater than 75 kW and less than 750 kW, greater than 100 kW and less than 500 kW, greater than 100 kW and less than 400 kW, greater than 200 kW and less than 300 kW, or greater than 100 kW and less than 500 kW.

In various embodiments, a ratio in kW to ns of P_{P} to T_{P} can be greater than 1:5, greater than 1:3, greater than 1:1, greater than 2:1, greater than 3:1, greater than 5:1, greater than 10:1, or greater than 20:1.

In certain embodiments, the waveform can approximate a square wave.

In various embodiments, the waveform can approximate a triangular wave.

In some embodiments, the seed pulse can at least partially increase in amplitude over time.

The present invention may be used in a method for treating a treatment site within or adjacent to a vessel wall. The method can include one or more of the steps of positioning a distal end of an optical fiber within an inflatable balloon; delivering an energy pulse to the optical fiber with a laser that includes (i) a seed source that is configured to emit a seed pulse, and (ii) an amplifier that is configured to increase energy of the seed pulse, the energy pulse having a waveform with a duration T, a minimum power P₀, a peak power Pp, and a time from P₀ to P_{P} equal to T_{P}, wherein T_{P} is not greater than 40% of T; and the optical fiber emitting light energy in a direction away from the optical fiber upon receiving the energy pulse to generate a plasma pulse within the inflatable balloon.

The present invention is also directed toward a catheter system for treating a treatment site within or adjacent to a vessel wall. In various embodiments, the catheter system can include an inflatable balloon, an optical fiber and a laser. In some embodiments, the optical fiber can have a distal end that is positioned within the inflatable balloon. The optical fiber can be configured to receive an energy pulse so that the optical fiber emits light energy in a direction away from the optical fiber to generate a plasma pulse within the inflatable balloon. The laser can include (i) a seed source that is configured to emit a seed pulse that at least partially increases in amplitude over time, and (ii) an amplifier that is configured to increase energy of the seed pulse so that the laser generates the energy pulse that is received by the optical fiber. In certain embodiments, the energy pulse can have a waveform that approximates one of (i) a square wave, and (ii) a triangular wave. The waveform can have a duration T, a minimum power P₀, a peak power P_{P}, and a time from P₀ to P_{P} equal to T_{P}. In some embodiments, T_{P} is not greater than 40% of T, T is within the range of greater than 50 ns and less than 3 µs, T_{P} is within the range of greater than 2.5 ns and less than 1 µs, P_{P} is within the range of greater than 50 kW and less than 1000 kW, and a ratio in kW to ns of P_{P} to T_{P} is greater than 1:2.

The present invention may also be used for treating a treatment site within or adjacent to a vessel wall or a heart valve. The method may include the steps of positioning a fiber distal end of an optical fiber within an inflatable balloon; delivering an energy pulse to the optical fiber with a laser that includes (i) a seed source that is configured to emit a seed pulse, and (ii) an amplifier that is configured to increase energy of the seed pulse, the energy pulse having a waveform with a duration T, a minimum power P₀, a peak power Pp, and a time from P₀ to P_{P} equal to T_{P}, wherein T_{P} is not greater than 40% of T; and the optical fiber emitting light energy in a direction away from the optical fiber upon receiving the energy pulse to generate a plasma pulse within the inflatable balloon.

The present invention is also directed toward a catheter system for treating a treatment site within or adjacent to a vessel wall or a heart valve. In certain embodiments, the catheter system includes an inflatable balloon, an optical fiber and a laser. The optical fiber has a fiber distal end positioned within the inflatable balloon. The optical fiber can be configured to receive an energy pulse so that the optical fiber emits light energy in a direction away from the optical fiber to generate a plasma pulse within the inflatable balloon. The laser can include (i) a seed source that is configured to emit a seed pulse, and (ii) an amplifier that is configured to increase energy of the seed pulse so that the laser generates the energy pulse that is received by the optical fiber. The energy pulse can have a waveform that approximates a square wave or a triangular wave. In various embodiments, the waveform can have a duration T, a minimum power P₀, a peak power Pp, and a time from P₀ to P_{P} equal to T_{P}. In certain embodiments, T_{P} can be greater than 40% of T.

The present invention may also be used for treating a treatment site within or adjacent to a vessel wall or a heart valve. The method can include the steps of positioning a fiber distal end of an optical fiber within an inflatable balloon; delivering an energy pulse to the optical fiber with a laser that includes (i) a seed source that is configured to emit a seed pulse, and (ii) an amplifier that is configured to increase energy of the seed pulse, the energy pulse having a waveform that approximates a square wave or a triangular wave, the waveform having a duration T, a minimum power P₀, a peak power Pp, and a time from P₀ to P_{P} equal to T_{P}, wherein T_{P} is greater than 40% of T; and the optical fiber emitting light energy in a direction away from the optical fiber upon receiving the energy pulse to generate a plasma pulse within the inflatable balloon.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope herein is defined by the appended claims.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Figure 1 is a schematic cross-sectional view of a catheter system having features of the present invention in accordance with various embodiments herein;
Figure 2 is a graph illustrating curves representative of three types of energy pulses, including a Gaussian energy pulse, a first square pulse at Full Width Half Max (FWHM) and a second square pulse at Full Width 20% Max;
Figure 3 is a graph illustrating a low-power seed pulse generated by a seed source of a laser;
Figure 4 is a graph illustrating one embodiment of an energy pulse generated by the laser, the energy pulse having a somewhat square wave pulse shape;
Figure 5 is a graph illustrating another embodiment of an energy pulse generated by the laser, the energy pulse having a somewhat triangular wave pulse shape; and
Figure 6 is a graph illustrating another embodiment of an energy pulse generated by the laser, the energy pulse having a somewhat different triangular wave pulse shape than that illustrated in Figure 5.

While embodiments are susceptible to various modifications and alternative forms, specifics thereof have been shown by way of example and drawings, and will be described in detail. It should be understood, however, that the scope herein is not limited to the particular aspects described.

### DESCRIPTION

Treatment of vascular lesions can reduce major adverse events or death in affected subjects. A major adverse event is one that can occur anywhere within the body due to the presence of a vascular lesion. Major adverse events can include, but are not limited to major adverse cardiac events, major adverse events in the peripheral or central vasculature, major adverse events in the brain, major adverse events in the musculature, or major adverse events in any of the internal organs.

As used herein, the treatment site can include a vascular lesion such as a calcified vascular lesion or a fibrous vascular lesion (hereinafter sometimes referred to simply as a "lesion"), typically found in a blood vessel and/or a heart valve. Plasma formation can initiate a pressure wave and can initiate the rapid formation of one or more bubbles that can rapidly expand to a maximum size and then dissipate through a cavitation event that can also launch a pressure wave upon collapse. The rapid expansion of the plasma-induced bubbles can generate one or more pressure waves within a balloon fluid and thereby impart pressure waves upon the treatment site. The pressure waves can transfer mechanical energy through an incompressible balloon fluid to a treatment site to impart a fracture force on the lesion. Without wishing to be bound by any particular theory, it is believed that the rapid change in balloon fluid momentum upon a balloon wall of the inflatable balloon that is in contact with or positioned near the lesion is transferred to the lesion to induce fractures in the lesion.

Those of ordinary skill in the art will realize that the following detailed description of the present invention is illustrative only and is not intended to be in any way limiting. Other embodiments of the present invention will readily suggest themselves to such skilled persons having the benefit of this disclosure. Additionally, other methods of delivering energy to the lesion can be utilized, including, but not limited to, electric current induced plasma generation. Reference will now be made in detail to implementations of the present invention as illustrated in the accompanying drawings.

In the interest of clarity, not all of the routine features of the implementations described herein are shown and described. It will, of course, be appreciated that in the development of any such actual implementation, numerous implementation-specific decisions must be made in order to achieve the developer's specific goals, such as compliance with application-related and business-related constraints, and that these specific goals will vary from one implementation to another and from one developer to another. Moreover, it is appreciated that such a development effort might be complex and time-consuming, but would nevertheless be a routine undertaking of engineering for those of ordinary skill in the art having the benefit of this disclosure.

As used herein, the terms "intravascular lesion", "vascular lesion" and "treatment site" are used interchangeably unless otherwise noted, and can include lesions located at or near blood vessels or heart valves.

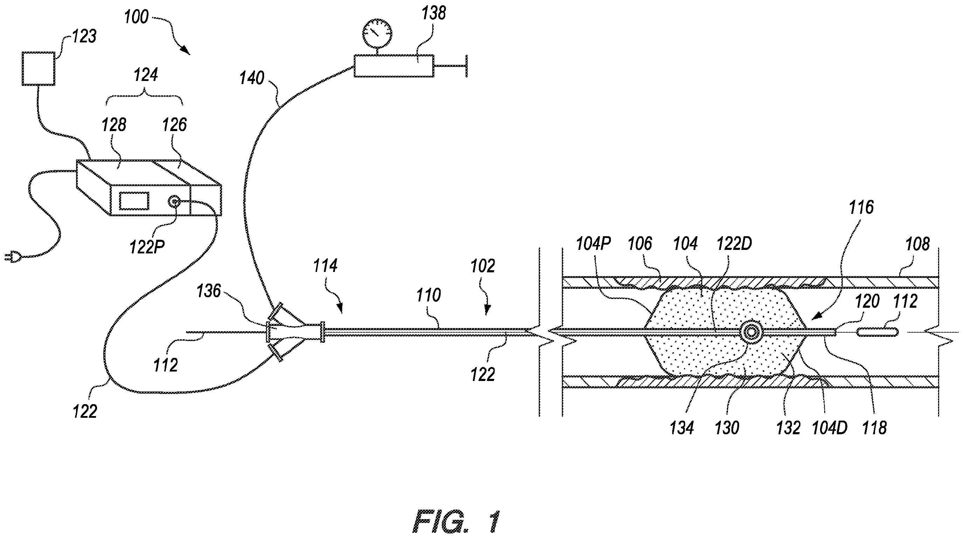

It is appreciated that the catheter systems herein can include many different forms and/or configurations other than those specifically shown and/or described herein. Referring now to Figure 1, a schematic cross-sectional view is shown of a catheter system in accordance with various embodiments herein. A catheter system 100 is suitable for imparting pressure to induce fractures in a vascular lesion within or adjacent a vessel wall of a blood vessel and/or a heart valve. In the embodiment illustrated in Figure 1, the catheter system 100 can include one or more of a catheter 102, one or more optical fibers 122, a controller 123, a laser 124, a manifold 136 and a fluid pump 138.

The catheter 102 includes an inflatable balloon 104 (sometimes referred to herein as "balloon"). The catheter 102 is configured to move to a treatment site 106 within or adjacent to a blood vessel 108 or a heart valve. The treatment site 106 can include a vascular lesion such as a calcified vascular lesion, for example. Additionally, or in the alternative, the treatment site 106 can include a vascular lesion such as a fibrous vascular lesion.

The catheter 102 can include the balloon 104, a catheter shaft 110 and a guidewire 112. The balloon can be coupled to the catheter shaft 110. The balloon can include a balloon proximal end 104P and a balloon distal end 104D. The catheter shaft 110 can extend between a shaft proximal end 114 and a shaft distal end 116. The catheter shaft 110 can include a guidewire lumen 118 which is configured to move over the guidewire 112. The catheter shaft 110 can also include an inflation lumen (not shown). In some embodiments, the catheter 102 can have a distal end opening 120 and can accommodate and be moved over and/or along the guidewire 112 so that the balloon 104 is positioned at or near the treatment site 106.

The balloon 104 can include a balloon wall 130. The balloon 104 can expand from a collapsed configuration suitable for advancing at least a portion of the catheter shaft 102 through a patient's vasculature to an expanded configuration suitable for anchoring the catheter 102 into position relative to the treatment site 106.

The catheter shaft 110 of the catheter 102 can encircle one or more optical fibers 122 (only one optical fiber 122 is illustrated in Figure 1 for clarity) in optical communication with a laser 124. The optical fiber 122 can be at least partially disposed along and/or within the catheter shaft 110 and at least partially within the balloon 104. In some embodiments, the catheter shaft 110 can encircle multiple optical fibers 122 such as a second optical fiber, a third optical fiber, etc.

The optical fiber 122 has a fiber proximal end 122P that is positioned at or adjacent to the laser 124, and a fiber distal end 122D that is positioned within the inflatable balloon 104. The optical fiber 122 extends between the laser 124 and the balloon 104. The optical fiber 122 is in optical communication with the laser 124.

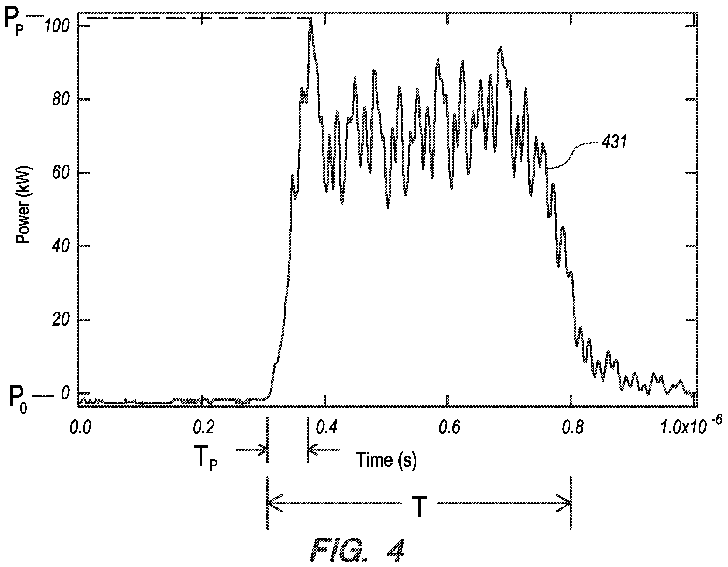

The controller 123 can control the laser 124 so that the laser 124 can generate one or more energy pulses 431 (illustrated in Figure 4, for example) as provided in greater detail herein. The controller 123 may also perform other relevant functions to control operation of the catheter 102.

The laser 124 of the catheter system 100 can be configured to provide one or more sub-millisecond energy pulses 431 that are sent to and received by the optical fiber 122. The optical fiber 122 acts as a conduit for light energy that is generated by the energy pulse(s) 431. In certain embodiments, the laser 124 can include one or more seed sources 126 and one or more amplifiers 128. Each amplifier 128 can be in optical communication with at least one of the seed sources 126. The seed source(s) 126 can each emit a relatively low-power seed pulse that is received and amplified by the amplifier 128. The amplifier 128 can increase the power of the seed pulse to generate the energy pulse 431. In one embodiment, the laser 124 can include one seed source 126 and one amplifier 128. Alternatively, the laser 124 can include a plurality of seed sources 126 and one amplifier 128. Still alternatively, the laser 124 can include a plurality of seed sources 126 and a plurality of amplifiers 128.

The light energy that is generated by the energy pulse(s) 431 is delivered by the optical fiber 122 to a location within the balloon 104. The light energy induces plasma formation in the form of a plasma pulse 134 that occurs in the balloon fluid 132 within the balloon 104. The plasma pulse 134 causes rapid bubble formation, and imparts pressure waves upon the treatment site 106. Exemplary plasma pulses 134 are shown in Figure 1. The balloon fluid 132 can be a liquid or a gas. As provided in greater detail herein, the plasma-induced bubbles 134 are intentionally formed at some distance away from the optical fiber 122 so that the likelihood of damage to the optical fiber is decreased.

In various embodiments, the sub-millisecond pulses of light can be delivered to near the treatment site 106 at a frequency of from at least approximately 1 hertz (Hz) up to approximately 5000 Hz. In some embodiments, the sub-millisecond pulses of light can be delivered to near the treatment site 106 at a frequency from at least 30 Hz to 1000 Hz. In other embodiments, the sub-millisecond pulses of light can be delivered to near the treatment site 106 at a frequency from at least 10 Hz to 100 Hz. In yet other embodiments, the sub-millisecond pulses of light can be delivered to near the treatment site 106 at a frequency from at least 1 Hz to 30 Hz.

It is appreciated that the catheter system 100 herein can include any number of optical fibers 122 in optical communication with the laser 124 at the proximal portion 114, and with the balloon fluid 132 within the balloon 104 at the distal portion 116. For example, in some embodiments, the catheter system 100 herein can include 1-30 optical fibers 122. In some embodiments, the catheter system 100 herein can include greater than 30 optical fibers.

The manifold 136 can be positioned at or near the shaft proximal end 114. The manifold 136 can include one or more proximal end openings that can receive the one or more optical fibers, such as optical fiber 122, the guidewire 112, and/or an inflation conduit 140. The catheter system 100 can also include the fluid pump 138 that is configured to inflate the balloon 104 with the balloon fluid 132 and/or deflate the balloon 104 as needed.

As with all embodiments illustrated and described herein, various structures may be omitted from the figures for clarity and ease of understanding. Further, the figures may include certain structures that can be omitted without deviating from the intent and scope of the invention.

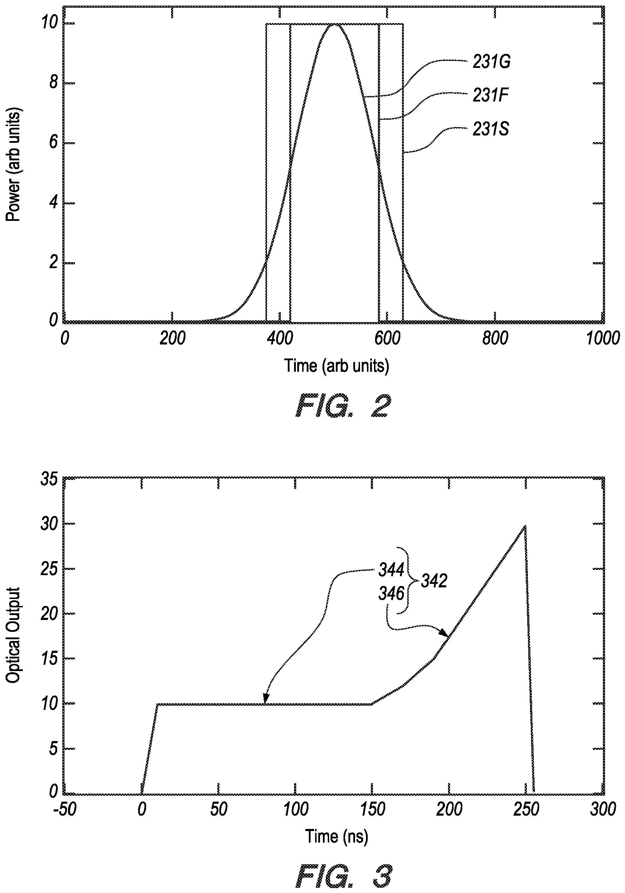

Figure 2 is a graph illustrating curves representative of three types of energy pulses, including a Gaussian energy pulse 231G, a first square energy pulse 231F at Full Width Half Max (FWHM) and a second square energy pulse 231S at Full Width 20% Max. The amount of energy that can be transmitted through the optical fiber 122 (illustrated in Figure 1) is limited by the peak intensity of the energy pulse on the fiber proximal end 122P (illustrated in Figure 1) of the optical fiber 122.

In this embodiment, using the first square wave 231F with the same peak amplitude of the Gaussian energy pulse 231G and a pulse width equal to the FWHM of the Gaussian energy pulse 231G puts at least approximately 23% more power into the optical fiber 122. Since plasma initiation typically occurs at 20% or less of this peak value, and the second square energy pulse 231S can be scaled to equal time above threshold, at least 54% more energy can be put through the optical fiber 122. In an alternative embodiment, similar or equivalent energy can be put through the optical fiber 122 in the same time at a lower peak power, allowing operation further below the absolute damage threshold of the optical fiber 122.

The optically generated plasma events that produce the desired pressure waves generally have an initiation threshold below (<20%) the damage threshold of the optical fiber 122. A faster rise time on the optical pulse (approaching a square wave), can allow more energy to be pumped into the plasma and bubble while both have minimal spatial extent. This can more efficiently pump energy into the pressure pulse and can thereby allow the generation of larger pressure gradients while using a lower optical pulse energy. Consequently, sufficiently energetic pressure waves can potentially be created to fracture calcified lesions while remaining well below the damage threshold of the optical fiber 122.

In one embodiment, a MOPA (Master Oscillator - Power Amplifier) can be used to produce the required pulse energy, beam quality, and pulse length. With this design, a well-controlled seed source 126, such as a low-power master oscillator, can seed the amplifier 128 (illustrated in Figure 1) with an appropriate pulse to amplify. As long as the pulse energy is low enough that the amplifier gain is not significantly depleted during the pulse, the temporal shape of the output of the system will closely match the output of the seed source 126. However, this can limit the amount of energy that can be extracted from the amplifier 128, increasing the size, cost, energy usage, and cooling requirements of the system.

Figure 3 is a graph illustrating one embodiment of a low-power seed pulse 342 generated by the seed source 126 (illustrated in Figure 1) of the laser 124 (illustrated in Figure 1). An alternative to running the amplifier 128 (illustrated in Figure 1) in the un-depleted gain regime is to pre-distort the seed pulse 342 of the seed source 126 to compensate for the distortions imparted by the amplifier 128. In general, since the gain in the amplifier 128 will decrease over the length of the seed pulse 342, the seed pulse 342 can have a lower amplitude toward a pulse beginning 344 of the seed pulse 342 than at a pulse end 346 of the seed pulse 342, as illustrated in Figure 3. Stated another way, the seed pulse 342 can increase in amplitude over time. Alternatively, the seed pulse 342 can remain substantially constant over time. Still alternatively, the seed pulse 342 can decrease over time. In yet another embodiment, the seed pulse 342 can include one or more increases and/or decreases over time.

The shape of the seed pulse 342 illustrated in the embodiment shown in Figure 3 could be appropriate for an amplifier 128 having enough stored energy to remain in an unsaturated gain regime for approximately 150 ns. It is understood that seed pulses could be appropriately tailored for an amplifier 128 having enough stored energy to remain in an unsaturated gain regime for longer or shorter durations than 150 ns. In the representative embodiment illustrated in Figure 3, the inversion in the amplifier 128 will have been reduced enough to reduce the gain available for the seed pulse 342, so increasing the power of the pulse end 346 of the seed pulse 342 can keep the output power of the energy pulse 231F, 231S (illustrated in Figure 2) more constant over the remaining portion of the energy pulse 231F, 231S.

Figure 4 is a graph illustrating one embodiment of an energy pulse 431 generated by the laser 124 (illustrated in Figure 1). In this embodiment, the energy pulse 431 approximates a square wave pulse shape. In certain such embodiments, the energy pulse 431 has a waveform having a duration T, a minimum power P₀, a peak power P_{P}, and a time from P₀ to P_{P} equal to T_{P}, such that T_{P} is less than 40% of T. In the specific embodiment illustrated in Figure 4, T is approximately 500 ns, and T_{P} is approximately 75 ns. Therefore, in this embodiment, T_{P} is approximately 15% of T. In non-exclusive alternative embodiments, T_{P} can be less than 30%, 25%, 20%, 10% or 5% of T. In still other embodiments, T_{P} can be greater than 40% of T. For example, in various embodiments, T_{P} can be greater than 40%, 50%, 60%, 75% or 90% of T, provided the energy pulse approximates a square wave pulse shape.

In the embodiment illustrated in Figure 4, T of the energy pulse 431 can be within the range of greater than 50 ns and less than 3 µs. In non-exclusive alternative embodiments, T can be within the range of greater than 100 ns and less than 2 µs, within the range of greater than 200 ns and less than 1 µs, within the range of greater than 300 ns and less than 800 ns, or within the range of greater than 400 ns and less than 600 ns. Still alternatively, T can have a duration within or outside of the foregoing ranges.

In this embodiment, T_{P} of the energy pulse 431 can have a duration within the range of greater than 2.5 ns and less than 1 µs. In non-exclusive alternative embodiments, T_{P} can have a duration within the range of greater than 5 ns and less than 800 ns, within the range of greater than 10 ns and less than 400 ns, within the range of greater than 15 ns and less than 300 ns, or within the range of greater than 30 ns and less than 100 ns. Still alternatively, T_{P} can have a duration within or outside of the foregoing ranges.

In the embodiment illustrated in Figure 4, the energy pulse 431 has a P_{P} of approximately 100 kW. P_{P} is within the range of greater than 50 kW and less than 1000 kW, preferably within the range of greater than 75 kW and less than 750 kW, within the range of greater than 100 kW and less than 500 kW, within the range of greater than 100 kW and less than 400 kW, or within the range of greater than 200 kW and less than 300 kW.

In this embodiment, the energy pulse 431 can have a ratio of P_{P} to T_{P} (in kW to ns) that is greater than 1:5. In non-exclusive alternative embodiments, the energy pulse 431 can have a ratio of P_{P} to T_{P} (in kW to ns) that is greater than 1:3, 1:1, 2:1, 3:1, 5:1, 10:1, or 20:1.

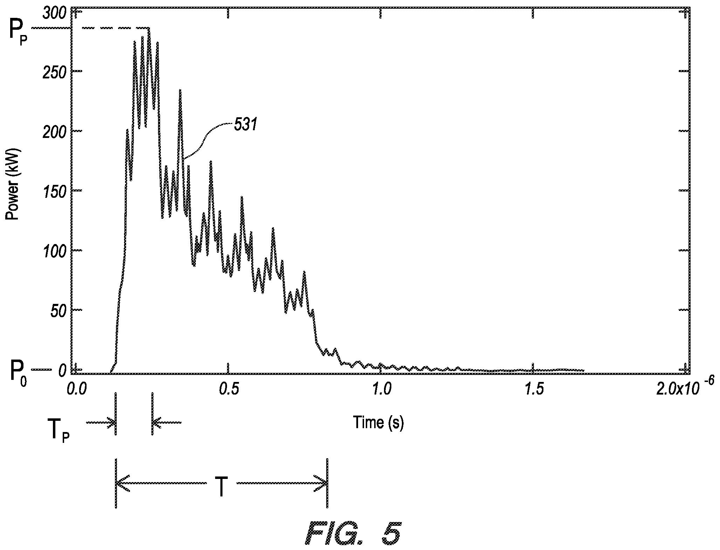

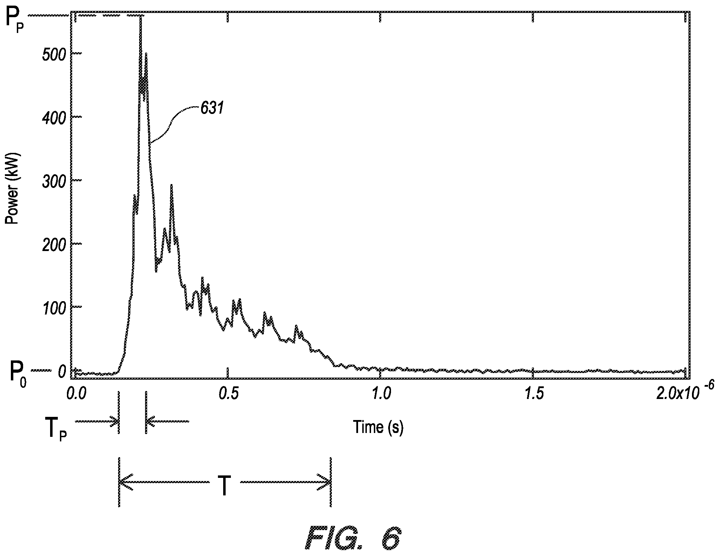

Figure 5 is a graph illustrating one embodiment of an energy pulse 531 generated by the laser 124 (illustrated in Figure 1). In this embodiment, the energy pulse 531 approximates a triangular wave pulse shape. Further, in this embodiment, the energy pulse 531 has a waveform having a duration T, a minimum power P₀, a peak power P_{P}, and a time from P₀ to P_{P} equal to T_{P}, such that T_{P} is less than 40% of T. In the specific embodiment illustrated in Figure 6, T is approximately 600 ns, and T_{P} is approximately 120 ns. Therefore, in this embodiment, T_{P} is approximately 20% of T. In non-exclusive alternative embodiments, T_{P} can be less than 40%, 30%, 25%, 15%, 10% or 5% of T. In still other embodiments, T_{P} can be greater than 40% of T. For example, in various embodiments, T_{P} can be greater than 40%, 50%, 60%, 75% or 90% of T, provided the energy pulse approximates a triangular wave pulse shape.

In the embodiment illustrated in Figure 5, T of the energy pulse 531 can be within the range of greater than 50 ns and less than 3 µs. In non-exclusive alternative embodiments, T can be within the range of greater than 100 ns and less than 2 µs, within the range of greater than 200 ns and less than 1 µs, within the range of greater than 300 ns and less than 800 ns, or within the range of greater than 400 ns and less than 600 ns, Still alternatively, T can have a duration within or outside of the foregoing ranges.

In this embodiment, T_{P} of the energy pulse 531 can have a duration within the range of greater than 2.5 ns and less than 1 µs. In non-exclusive alternative embodiments, T_{P} can have a duration within the range of greater than 5 ns and less than 800 ns, within the range of greater than 10 ns and less than 400 ns, within the range of greater than 15 ns and less than 300 ns, or within the range of greater than 30 ns and less than 100 ns. Still alternatively, T_{P} can have a duration within or outside of the foregoing ranges.

In the embodiment illustrated in Figure 5, the energy pulse 531 has a P_{P} of approximately 280 kW. P_{P} is within the range of greater than 50 kW and less than 1000 kW, preferably within the range of greater than 75 kW and less than 750 kW, within the range of greater than 100 kW and less than 500 kW, within the range of greater than 100 kW and less than 400 kW, or within the range of greater than 200 kW and less than 300 kW.

In this embodiment, the energy pulse 531 can have a ratio of P_{P} to T_{P} (in kW to ns) that is greater than 1:5. In non-exclusive alternative embodiments, the energy pulse 531 can have a ratio of P_{P} to T_{P} (in kW to ns) that is greater than 1:3, 1:1, 2:1, 3:1, 5:1, 10:1, or 20:1.

Figure 6 is a graph illustrating one embodiment of an energy pulse 631 generated by the laser 124 (illustrated in Figure 1). In this embodiment, the energy pulse 631 approximates a somewhat more pronounced triangular wave pulse shape from that illustrated in Figure 5. Further, in this embodiment, the energy pulse 631 has a waveform having a duration T, a minimum power P₀, a peak power P_{P}, and a time from P₀ to P_{P} equal to T_{P}, such that T_{P} is less than 40% of T. In the specific embodiment illustrated in Figure 6, T is approximately 600 ns, and T_{P} is approximately 75 ns. Therefore, in this embodiment, T_{P} is approximately 12.5% of T. In non-exclusive alternative embodiments, T_{P} can be less than 40%, 30%, 25%, 20%, 15%, 10% or 5% of T. In still other embodiments, T_{P} can be greater than 40% of T. For example, in various embodiments, T_{P} can be greater than 40%, 50%, 60%, 75% or 90% of T, provided the energy pulse approximates a pronounced triangular wave pulse shape.

In the embodiment illustrated in Figure 6, T of the energy pulse 631 can be within the range of greater than 50 ns and less than 3 µs. In non-exclusive alternative embodiments, T can be within the range of greater than 100 ns and less than 2 µs, within the range of greater than 200 ns and less than 1 µs, within the range of greater than 300 ns and less than 800 ns, or within the range of greater than 400 ns and less than 600 ns, Still alternatively, T can have a duration within or outside of the foregoing ranges.

In this embodiment, T_{P} of the energy pulse 631 can have a duration within the range of greater than 2.5 ns and less than 1 µs. In non-exclusive alternative embodiments, T_{P} can have a duration within the range of greater than 5 ns and less than 800 ns, within the range of greater than 10 ns and less than 400 ns, within the range of greater than 15 ns and less than 300 ns, or within the range of greater than 30 ns and less than 100 ns. Still alternatively, T_{P} can have a duration within or outside of the foregoing ranges.

In the embodiment illustrated in Figure 6, the energy pulse 631 has a P_{P} is approximately 550 kW. is within the range of greater than 50 kW and less than 1000 kW, preferably within the range of greater than 75 kW and less than 750 kW, within the range of greater than 100 kW and less than 500 kW, within the range of greater than 100 kW and less than 400 kW, or within the range of greater than 200 kW and less than 300 kW.

In this embodiment, the energy pulse 631 can have a ratio of P_{P} to T_{P} (in kW to ns) that is greater than 1:5. In non-exclusive alternative embodiments, the energy pulse 631 can have a ratio of P_{P} to T_{P} (in kW to ns) that is greater than 1:3, 1:1, 2:1, 3:1, 5:1, 10:1, or 20:1.

It is recognized that any temporal energy pulse shape that features a relatively fast rise time and attempts to decrease the overshot on the leading edge of the energy pulse should allow more efficient plasma generation thorough an optical fiber without damaging the optical fiber. For example, any temporal energy pulse shape which features a faster rise (and fall) than a Gaussian could reap the benefits of this invention.

The present invention is also used for treating a treatment site within or adjacent to a vessel wall, with such methods utilizing the devices disclosed herein.

### Lasers

The lasers suitable for use herein can include various types of lasers including lasers and lamps. Suitable lasers can include short pulse lasers on the sub-millisecond timescale. In some embodiments, the laser can include lasers on the nanosecond (ns) timescale. The lasers can also include short pulse lasers on the picosecond (ps), femtosecond (fs), and microsecond (us) timescales. It is appreciated that there are many combinations of laser wavelengths, pulse widths and energy levels that can be employed to achieve plasma in the balloon fluid of the catheters illustrated and/or described herein. In various embodiments, the pulse widths can include those falling within a range including from at least 10 ns to 200 ns. In some embodiments, the pulse widths can include those falling within a range including from at least 20 ns to 100 ns. In other embodiments, the pulse widths can include those falling within a range including from at least 1 ns to 5000 ns.

Exemplary nanosecond lasers can include those within the UV to IR spectrum, spanning wavelengths of about 10 nanometers to 1 millimeter. In some embodiments, the lasers suitable for use in the catheter systems herein can include those capable of producing light at wavelengths of from at least 750 nm to 2000 nm. In some embodiments, the lasers can include those capable of producing light at wavelengths of from at least 700 nm to 3000 nm. In some embodiments, the lasers can include those capable of producing light at wavelengths of from at least 100 nm to 10 micrometers (µm). Nanosecond lasers can include those having repetition rates of up to 200 kHz. In some embodiments, the laser can include a Q-switched thulium:yttrium-aluminum-garnet (Tm:YAG) laser. In some embodiments, the laser can include a neodymium:yttrium-aluminum-garnet (Nd:YAG), holmium:yttrium-aluminum-garnet (Ho:YAG), erbium:yttrium-aluminum-garnet (Er:YAG), excimer laser, helium-neon laser, carbon dioxide laser, as well as doped, pulsed, fiber lasers.

### Pressure Waves

The catheters illustrated and/or described herein can generate pressure waves having maximum pressures in the range of at least 1 megapascal (MPa) to 100 MPa. The maximum pressure generated by a particular catheter will depend on the laser, the absorbing material, the bubble expansion, the propagation medium, the balloon material, and other factors. In some embodiments, the catheters illustrated and/or described herein can generate pressure waves having maximum pressures in the range of at least 2 MPa to 50 MPa. In other embodiments, the catheters illustrated and/or described herein can generate pressure waves having maximum pressures in the range of at least 2 MPa to 30 MPa. In yet other embodiments, the catheters illustrated and/or described herein can generate pressure waves having maximum pressures in the range of at least 15 MPa to 25 MPa. In some embodiments, the catheters illustrated and/or described herein can generate pressure waves having peak pressures of greater than or equal to 1 MPa, 2 MPa, 3 MPa, 4 MPa, 5 MPa, 6 MPa, 7 MPa, 8 MPa, 9 MPa, 10 MPa, 11 MPa, 12 MPa, 13 MPa, 14 MPa, 15 MPa, 16 MPa, 17 MPa, 18 MPa, 19 MPa, 20 MPa, 21 MPa, 22 MPa, 23 MPa, 24 MPa, or 25 MPa, 26 MPa, 27 MPa, 28 MPa, 29 MPa, 30 MPa, 31 MPa, 32 MPa, 33 MPa, 34 MPa, 35 MPa, 36 MPa, 37 MPa, 38 MPa, 39 MPa, 40 MPa, 41 MPa, 42 MPa, 43 MPa, 44 MPa, 45 MPa, 46 MPa, 47 MPa, 48 MPa, 49 MPa, or 50 MPa,. It is appreciated that the catheters illustrated and/or described herein can generate pressure waves having operating pressures or maximum pressures that can fall within a range, wherein any of the forgoing numbers can serve as the lower or upper bound of the range, provided that the lower bound of the range is a value less than the upper bound of the range.

Therapeutic treatment can act via a fatigue mechanism or a brute force mechanism. For a fatigue mechanism, operating pressures would be about at least 0.5 MPa to 2 MPa, or about 1 MPa. For a brute force mechanism, operating pressures would be about at least 20 MPa to 30 MPa, or about 25 MPa. Pressures between the extreme ends of these two ranges may act upon a treatment site using a combination of a fatigue mechanism and a brute force mechanism.

The pressure waves described herein can be imparted upon the treatment site from a distance within a range from at least 0.01 millimeters (mm) to 25 mm extending radially from a longitudinal axis of a catheter placed at a treatment site. In some embodiments, the pressure waves can be imparted upon the treatment site from a distance within a range from at least 1 mm to 20 mm extending radially from a longitudinal axis of a catheter placed at a treatment site. In other embodiments, the pressure waves can be imparted upon the treatment site from a distance within a range from at least 0.1 mm to 10 mm extending radially from a longitudinal axis of a catheter placed at a treatment site. In yet other embodiments, the pressure waves can be imparted upon the treatment site from a distance within a range from at least 1.5 mm to 4 mm extending radially from a longitudinal axis of a catheter placed at a treatment site. In some embodiments, the pressure waves can be imparted upon the treatment site from a range of at least 2 MPa to 30 MPa at a distance from 0.1 mm to 10 mm. In some embodiments, the pressure waves can be imparted upon the treatment site from a range of at least 2 MPa to 25 MPa at a distance from 0.1 mm to 10 mm. In some embodiments, the pressure waves can be imparted upon the treatment site from a distance that can be greater than or equal to 0.1 mm, 0.2 mm, 0.3 mm, 0.4 mm, 0.5 mm, 0.6 mm, 0.7 mm, 0.8 mm, or 0.9 mm, 1 mm, 2 mm, 3 mm, 4 mm, 5 mm, 6 mm, 7 mm, 8 mm, 9 mm, or 10 mm, or can be an amount falling within a range between, or outside the range of any of the foregoing.

By shaping the temporal form of the optical pulse to have a fast rise time and minimal overshoot (ideally approaching a square wave), the efficiency for generating the pressure wave can be improved, and the amount of energy that can be delivered in a given time interval can be increased while decreasing the peak laser intensity to remain below the damage threshold of the optical fiber.

It should be noted that, as used in this specification and the appended claims, the singular forms "a," "an," and "the" include plural referents unless the content and/or context clearly dictates otherwise. It should also be noted that the term "or" is generally employed in its sense including "and/or" unless the content or context clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

As used herein, the recitation of numerical ranges by endpoints shall include all numbers subsumed within that range, inclusive (e.g., 2 to 8 includes 2, 2.1, 2.8, 5.3, 7, 8, etc.).

It is recognized that the figures shown and described are not necessarily drawn to scale, and that they are provided for ease of reference and understanding, and for relative positioning of the structures.

The headings used herein are provided for consistency with suggestions under 37 CFR 1.77 or otherwise to provide organizational cues. These headings shall not be viewed to limit or characterize the invention(s) set out in any claims that may issue from this disclosure. As an example, a description of a technology in the "Background" is not an admission that technology is prior art to any invention(s) in this disclosure. Neither is the "Summary" or "Abstract" to be considered as a characterization of the invention(s) set forth in issued claims.

The embodiments described herein are not intended to be exhaustive or to limit the invention to the precise forms disclosed in the following detailed description. Rather, the embodiments are chosen and described so that others skilled in the art can appreciate and understand the principles and practices. As such, aspects have been described with reference to various specific and preferred embodiments and techniques. However, it should be understood that many variations and modifications may be made while remaining within the scope of protection.

It is understood that although a number of different embodiments of the catheter systems have been illustrated and described herein, one or more features of any one embodiment can be combined with one or more features of one or more of the other embodiments, provided that such combination satisfies the intent of the present invention.

While a number of exemplary aspects and embodiments of the catheter systems have been discussed above, those of skill in the art will recognize certain modifications, permutations, additions and sub-combinations thereof.

## Claims

1. A catheter system (100) for treating a treatment site (106) within or adjacent to a vessel wall or a heart valve, the catheter system (100) comprising:
an inflatable balloon (104);
an optical fiber (122) having a fiber distal end (122D) positioned within the inflatable balloon (104), the optical fiber (122) being configured to receive an energy pulse (431) so that the optical fiber emits light energy in a direction away from the optical fiber (122) to generate a plasma pulse (134) within the inflatable balloon (104); and
a laser including (i) a seed source (126) that is configured to emit a seed pulse (342), and (ii) an amplifier (128) that is configured to increase energy of the seed pulse (342) so that the laser generates the energy pulse (431) that is received by the optical fiber (122), the energy pulse (431) having a waveform having a pulse duration (T), a minimum power (Po), a peak power (Pp) and a rise time (Tp) of increasing power from the minimum power (Po) to the peak power (Pp), the rise time (Tp) being not greater than 40% of the pulse duration (T);
**characterized in that** the peak power (Pp) of the energy pulse is within the range of greater than 50 kW and less than 1000 kW.

2. The catheter system (100) of claim 1 wherein the rise time (Tp) is not greater than 25% of the pulse duration (T).

3. The catheter system (100) of claim 1 wherein the rise time (Tp) is not greater than 10% of the pulse duration (T).

4. The catheter system (100) of any of claims 1-3 wherein the pulse duration (T) is within the range of greater than 50 ns and less than 3 µs.

5. The catheter system (100) of claim 4 wherein the pulse duration (T) is within the range of greater than 200 ns and less than 1 µs.

6. The catheter system (100) of claim 5 wherein the pulse duration (T) is within the range of greater than 400 ns and less than 600 ns.

7. The catheter system (100) of any of claims 4-6 wherein the rise time (Tp) is within the range of greater than 2.5 ns and less than 1 µs.

8. The catheter system (100) of claim 7 wherein the rise time (Tp) is within the range of greater than 10 ns and less than 400 ns.

9. The catheter system (100) of claim 8 wherein the rise time (Tp) is within the range of greater than 30 ns and less than 100 ns.

10. The catheter system (100) of any of claims 1-9 wherein the peak power (Pp) is within the range of greater than 100 kW and less than 400 kW.

11. The catheter system (100) of any of claims 1-10 wherein a ratio in kWto ns of the peak power (Pp) to the rise time (Tp) is greater than 1:5.

12. The catheter system (100) of claim 11 wherein a ratio in kW to ns the peak power (Pp) to the rise time (Tp) is greater than 5:1.

13. The catheter system (100) of any of claims 1-12 wherein the waveform approximates a square wave.

14. The catheter system (100) of any of claims 1-12 wherein the waveform approximates a triangular wave.

15. The catheter system (100) of any of claims 1-14 wherein the seed pulse increases in amplitude over time.

## Patentansprüche

1. Kathetersystem (100) zur Behandlung einer Behandlungsstelle (106) in oder neben einer Gefäßwand oder einer Herzklappe, wobei das Kathetersystem (100) umfasst:
einen aufblasbaren Ballon (104);
eine Lichtleitfaser (122) mit einem distalen Faserende (122D), das im aufblasbaren Ballon (104) positioniert ist, wobei die Lichtleitfaser (122) ausgelegt ist, um einen Energiepuls (431) zu empfangen, sodass die Lichtleitfaser Lichtenergie in eine Richtung weg von der Lichtleitfaser (122) emittiert, um einen Plasmapuls (134) im aufblasbaren Ballon (104) zu erzeugen; und
einen Laser, der (i) eine Seed-Quelle (126), die ausgelegt ist, um einen Seed-Puls (342)zu emittieren, und (ii) einen Verstärker (128) umfasst, der ausgelegt ist, um die Energie des Seed-Pulses (342) zu verstärken, sodass der Laser den Energiepuls (431) erzeugt, der vom Lichtleitfaser (122) empfangen wird, wobei der Energiepuls (431) eine Wellenform mit einer Pulsdauer (T), einer Mindestleistung (Po), einer Spitzenleistung (Pp) und einer Anstiegszeit (Tp) für die Erhöhung der Leistung von der Mindestleistung (Po) auf die Spitzenleistung (Pp) aufweist, wobei die Anstiegszeit (Tp) nicht mehr als 40 % der Pulsdauer (T) beträgt;
**dadurch gekennzeichnet, dass**
die Spitzenleistung (Pp) des Energiepulses im Bereich von mehr als 50 kW bis weniger als 1000 kW liegt.

2. Kathetersystem (100) nach Anspruch 1, wobei die Anstiegszeit (Tp) nicht mehr als 25 % der Pulsdauer (T) beträgt.

3. Kathetersystem (100) nach Anspruch 1, wobei die Anstiegszeit (Tp) nicht mehr als 10 % der Pulsdauer (T) beträgt.

4. Kathetersystem (100) nach einem der Ansprüche 1-3, wobei die Pulsdauer (T) im Bereich von mehr als 50 ns bis weniger als 3 µs liegt.

5. Kathetersystem (100) nach Anspruch 4, wobei die Pulsdauer (T) im Bereich von mehr als 200 ns bis weniger als 1 µs liegt.

6. Kathetersystem (100) nach Anspruch 5, wobei die Pulsdauer (T) im Bereich von mehr als 400 ns bis weniger als 600 ns liegt.

7. Kathetersystem (100) nach einem der Ansprüche 4-6, wobei die Anstiegszeit (Tp) im Bereich von mehr als 2,5 ns bis weniger als 1 µs liegt.

8. Kathetersystem (100) nach Anspruch 7, wobei die Anstiegszeit (Tp) im Bereich von mehr als 10 ns bis weniger als 400 ns liegt.

9. Kathetersystem (100) nach Anspruch 8, wobei die Anstiegszeit (Tp) im Bereich von mehr als 30 ns bis weniger als 100 ns liegt.

10. Kathetersystem (100) nach einem der Ansprüche 1-9, wobei die Spitzenleistung (Pp) im Bereich von mehr als 100 kW bis weniger als 400 kW liegt.

11. Kathetersystem (100) nach einem der Ansprüche 1-10, wobei das Verhältnis in kW zu ns der Spitzenleistung (Pp) zur Anstiegszeit (Tp) höher als 1:5 ist.

12. Kathetersystem (100) nach Anspruch 11, wobei das Verhältnis in kW zu ns der Spitzenleistung (Pp) zur Anstiegszeit (Tp) höher als 5:1 ist.

13. Kathetersystem (100) nach einem der Ansprüche 1-12, wobei sich die Wellenform einer Rechteckwelle annähert.

14. Kathetersystem (100) nach einem der Ansprüche 1-12, wobei sich die Wellenform einer Dreieckwelle annähert.

15. Kathetersystem (100) nach einem der Ansprüche 1-14, wobei die Amplitude des Seed-Pulses im Laufe der Zeit ansteigt.

## Revendications

1. Système de cathéter (100) pour traiter un site de traitement (106) à l'intérieur de ou adjacent à une paroi vasculaire ou une valvule cardiaque, le système de cathéter (100) comprenant :
un ballonnet gonflable (104) ;
une fibre optique (122) présentant une extrémité distale (122D) de fibre positionnée à l'intérieur du ballonnet gonflable (104), la fibre optique (122) étant configurée pour recevoir une impulsion d'énergie (431) de sorte que la fibre optique émette de l'énergie lumineuse dans une direction s'éloignant de la fibre optique (122) pour générer une impulsion de plasma (134) à l'intérieur du ballonnet gonflable (104) ; et
un laser comportant (i) une source initiale (126) qui est configurée pour émettre une impulsion initiale (342), et (ii) un amplificateur (128) qui est configuré pour accroître l'énergie de l'impulsion initiale (342) de sorte que le laser génère l'impulsion d'énergie (431) qui est reçue par la fibre optique (122), l'impulsion d'énergie (431) présentant une forme d'onde présentant une durée d'impulsion (T), une puissance minimale (Po), une puissance de crête (Pp) et un temps de montée (Tp) de puissance croissante de la puissance minimale (Po) à la puissance de crête (Pp), le temps de montée (Tp) étant inférieur ou égal à 40 % de la durée d'impulsion (T) ;
**caractérisé en ce que**
la puissance de crête (Pp) de l'impulsion d'énergie se situe dans la plage de plus de 50 kW et moins de 1 000 kW.

2. Système de cathéter (100) selon la revendication 1 dans lequel le temps de montée (Tp) est inférieur ou égal à 25 % de la durée d'impulsion (T).

3. Système de cathéter (100) selon la revendication 1 dans lequel le temps de montée (Tp) est inférieur ou égal à 10 % de la durée d'impulsion (T).

4. Système de cathéter (100) selon l'une quelconque des revendications 1 à 3 dans lequel la durée d'impulsion (T) se situe dans la plage de plus de 50 ns et moins de 3 µs.

5. Système de cathéter (100) selon la revendication 4 dans lequel la durée d'impulsion (T) se situe dans la plage de plus de 200 ns et moins de 1 µs.

6. Système de cathéter (100) selon la revendication 5 dans lequel la durée d'impulsion (T) se situe dans la plage de plus de 400 ns et moins de 600 ns.

7. Système de cathéter (100) selon l'une quelconque des revendications 4 à 6 dans lequel le temps de montée (Tp) se situe dans la plage de plus de 2,5 ns et moins de 1 µs.

8. Système de cathéter (100) selon la revendication 7 dans lequel le temps de montée (Tp) se situe dans la plage de plus de 10 ns et moins de 400 ns.

9. Système de cathéter (100) selon la revendication 8 dans lequel le temps de montée (Tp) se situe dans la plage de plus de 30 ns et moins de 100 ns.

10. Système de cathéter (100) selon l'une quelconque des revendications 1 à 9 dans lequel la puissance de crête (Pp) se situe dans la plage de plus de 100 kW et moins de 400 kW.

11. Système de cathéter (100) selon l'une quelconque des revendications 1 à 10 dans lequel un rapport en kW sur ns de la puissance de crête (Pp) sur le temps de montée (Tp) est supérieur à 1:5.

12. Système de cathéter (100) selon la revendication 11 dans lequel un rapport en kW sur ns de la puissance de crête (Pp) sur le temps de montée (Tp) est supérieur à 5:1.

13. Système de cathéter (100) selon l'une quelconque des revendications 1 à 12 dans lequel la forme d'onde ressemble à une onde carrée.

14. Système de cathéter (100) selon l'une quelconque des revendications 1 à 12 dans lequel la forme d'onde ressemble à une onde triangulaire.

15. Système de cathéter (100) selon l'une quelconque des revendications 1 à 14 dans lequel l'impulsion initiale augmente en amplitude au fil du temps.
